(19) Europäisches Patentamt / European Patent Office / Office européen des brevets

(11) **EP 1 994 949 A1**

(12) **EUROPEAN PATENT APPLICATION**
published in accordance with Art. 158(3) EPC

(43) Date of publication:
**26.11.2008 Bulletin 2008/48**

(51) Int Cl.:
*A61L 27/00* [(2006.01)] *C12M 3/00* [(2006.01)]
*C12N 5/06* [(2006.01)] *D04H 1/72* [(2006.01)]

(21) Application number: **07738224.0**

(22) Date of filing: **05.03.2007**

(86) International application number:
**PCT/JP2007/054739**

(87) International publication number:
**WO 2007/102606 (13.09.2007 Gazette 2007/37)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **06.03.2006 JP 2006059393**

(71) Applicant: **TEIJIN LIMITED**
**Osaka-shi,**
**Osaka 541-0054 (JP)**

(72) Inventors:
• **FUKUTOMI, Chiaki**
**Hino-shi, Tokyo 1910065 (JP)**
• **KANEKO, Hiroaki**
**Hino-shi, Tokyo 1910065 (JP)**
• **KITAZONO, Eiichi**
**Hino-shi, Tokyo 1910065 (JP)**
• **KAYASHIMA, Mika**
**Hino-shi, Tokyo 1910065 (JP)**
• **NAKAYAMA, Miyuki**
**Hino-shi, Tokyo 1910065 (JP)**

(74) Representative: **Hallybone, Huw George et al**
**Carpmaels & Ransford**
**43-45 Bloomsbury Square**
**London WC1A 2RA (GB)**

(54) **SCAFFOLD MATERIAL**

(57)    To provide a scaffold having excellent mechanical strength and cell growth capability and is suitable for use as a cell culture medium or a prosthetic material.

The present invention relates to a scaffold composed of an assembly of fibers and having a 3-D structure consisting of two end faces and a side face, wherein

(1) the fibers are aligned in a plane direction;
(2) the fibers have a diameter of 0.05 to 50 $\mu$m;
(3) the fibers are essentially composed of a biocompatible polymer; and
(4) the scaffold has an apparent density of 95 to 350 kg/m$^3$.

Fig. 1

**Description**

Technical Field

The present invention relates to a scaffold used for cell growth. The present invention relates to a scaffold which is composed of an assembly of fibers and suitable for use as a prosthetic material or a cell culture medium.

Background of the Art

As an approach to the treatment of a greatly damaged living tissue, active researches into regenerative medicine for the reconstruction of an original living tissue by making use of the differentiation and proliferation of a cell are now under way. When a cell differentiates or grows in *vivo,* an extracellular matrix serves as a scaffold to construct a tissue. However, when a tissue is greatly damaged, it must be compensated for by an artificial or natural material until the cell itself produces a matrix. That is, a scaffold (prosthetic material) is an important factor for providing the optimum environment for the construction of a tissue. The requirements for this scaffold include 1) bioabsorption, 2) cell adhesion, 3) porosity and 4) mechanical strength. With a view to the creation of a material which satisfies all the above requirements, synthetic polymers (such as polyglycolic acid, polylactic acid and polycaprolactone), natural polymers (such as collagen, gelatin, elastin, hyaluronic acid, alginic acid and chitosan), inorganic materials (such as hydroxylapatite and tricalcium β-phosphate) and composites thereof have been studied up till now.

As described above, porosity is one of the important requirements for the scaffold (prosthetic material). This is important so as to supply sufficient oxygen and nutrition which are required for the regeneration of a tissue and discharge carbon dioxide or waste materials quickly. Therefore, to attain the porosity of a scaffold, freeze-drying, phase separation and foaming techniques are proposed. As for a structure obtained by the freeze-drying or phase separation technique, the shape of each pore is isolated and the intrusion of a cell is difficult. Thus, the structure is unsatisfactory as a scaffold. A structure obtained by the foaming technique also has a problem that the intrusion of a cell is difficult because pores are isolated independently.

There is reported nonwoven cloth which is an assembly of fibers made of a thermoplastic polymer and having an average fiber diameter of 0.1 to 2 $\mu$m and an average apparent density of 10 to 95 kg/m$^3$, the arbitrary cross section of each fiber being irregular in shape (patent document 1). However, a scaffold which is thicker and stronger is desired.

There is also proposed a cartilage plug having a porous structure which is formed by preparing a polyurethane polymer containing a water-soluble substance such as saccharose and dissolving the water-soluble substance in a water bath (patent document 2). However, pores formed by this method are not continuous and there is limitation to cell growth.

It is also proposed to manufacture a scaffold by accumulating nanofibers in a plane by an electrospinning method and use it for the culture of a cell (non-patent document 1). This method has a defect that, when the fibers are accumulated to a predetermined thickness or more, an electrode is covered with the accumulated product as the fibers are collected on a planar collection electrode, whereby it is difficult to maintain a certain potential difference and the density of the accumulated fibers changes in the accumulation direction. The accumulation density of the accumulated product becomes nonuniform in a plane direction perpendicular to the accumulation direction. Therefore, to use this accumulated product as a scaffold for cell growth, the accumulation density must be made uniform to improve mechanical strength.

(patent document 1) WO2004/88024
(patent document 2) JP-A 2004-520855
(non-patent document 1) Published online 25 March 2002 in Wiley InterScience (www.interscience. wiley.com)

Disclosure of the Invention

It is an object of the present invention to provide a scaffold which is a high-density assembly of fibers and suitable for cell growth. It is another object of the present invention to provide a scaffold for cell growth which has excellent mechanical strength. It is still another object of the present invention to provide a scaffold which can grow a cell well. It is a further object of the present invention to provide a process of manufacturing the scaffold. It is a still further object of the present invention to provide a method of growing a cell by using the scaffold. It is a still further object of the present invention to provide a method of regenerating a living tissue by using the scaffold.

The inventors of the present invention have found that, when fibers are manufactured by an electrospinning method and accumulated on a rotary winding shaft, accumulated fibers having a uniform accumulation density in the accumulation direction are obtained.

They have also found that, when the fibers are accumulated on the rotary winding shaft, accumulated fibers which are uniform in a plane parallel to the winding shaft is obtained.

Further, they have found that, when the fibers are wound up on the rotary shaft, certain tension is applied to the fibers and high-density accumulated fibers are obtained.

They have also found that the obtained accumulated fibers have suitable strength and fiber density as a scaffold for cell growth. The present invention is based on these findings.

That is, the present invention is a scaffold which is composed of an assembly of fibers and has a 3-D structure consisting of two end faces and a side face, wherein

(1) the fibers are aligned in a plane direction,
(2) the fibers have a diameter of 0.05 to 50 $\mu$m,
(3) the fibers are essentially composed of a biocompatible polymer, and
(4) the scaffold has an apparent density of 95 to 350 kg/m$^3$.

The present invention is a process of manufacturing a scaffold, comprising the steps of:

(1) delivering a dope containing a biocompatible polymer into an electrostatic field formed between electrodes from a nozzle to form fibers;
(2) winding up the obtained fibers on a winding shaft to form a roll of the fibers which are aligned in a plane direction parallel to the winding shaft; and
(3) cutting out a 3-D structure from the obtained roll.

The present invention includes a method of dividing or growing a cell by using the scaffold. The present invention also includes a method of regenerating a living tissue by implanting the scaffold in a damaged affected part.

Brief Description of the Drawings

Fig. 1 shows an example of an apparatus used in an electrospinning method;
Fig. 2 shows another example of the apparatus used in the electrospinning method;
Fig. 3 shows still another example of the apparatus used in the electrospinning method;
Fig. 4 shows a further example of the apparatus used in the electrospinning method;
Fig. 5 shows a method of cutting out a scaffold in the manufacturing process of the present invention;
Fig. 6 shows a picture of the top end face of a scaffold obtained in Example 2;
Fig. 7 shows a picture of the bottom end face of the scaffold obtained in Example 2;
Fig. 8 shows a picture of the stained scaffold obtained in Example 2 on the 1st day of culture;
Fig. 9 shows a picture of the stained scaffold obtained in Example 2 on the 12th day of culture;
Fig. 10 shows a picture of the top end face of a scaffold obtained in Example 3;
Fig. 11 shows a picture of the bottom end face of the scaffold obtained in Example 3;
Fig. 12 shows a picture of the section of the scaffold obtained in Example 3;
Fig. 13 shows a picture of the stained scaffold obtained in Example 3 on the 1st day of culture; and
Fig. 14 shows a picture of the stained scaffold obtained in Example 3 on the 12th day of culture.

(explanation of letters or notations)

1. nozzle
2. dope
3. dope holding tank
4. positive electrode
5. negative electrode
6. high-voltage generator
7. winder
8. static electricity removing apparatus
9. winding shaft direction
10. scaffold cut out in direction parallel to winding shaft direction
11. direction perpendicular to winding direction
12. scaffold cut out in direction perpendicular to winding shaft

Best Mode for Carrying Out the Invention

The present invention will be described in detail hereinunder

<scaffold>

The scaffold of the present invention has a 3-D structure consisting of two end faces and a side face. The shape of each of the end faces is circular, elliptic, rectangular, etc. The end faces may be curved with irregularities. The two end faces may differ in shape and size. The area of each of the end faces is preferably 0.05 to 8 cm$^2$, more preferably 0.1 to 1 cm$^2$. The side face may be a continuous curved face or may consist of a plurality of faces. That is, the shape of the scaffold of the present invention is preferably a 3-D structure such as a cylinder or a polygonal column.

The scaffold of the present invention has a 3-D structure, that is, expanses in the transverse direction (x axis), longitudinal direction (y axis) and height direction (z axis). In this respect, it differs from plain nonwoven cloth having expanses in the transverse direction (x axis) and the longitudinal direction (y axis). In the scaffold of the present invention, the term "height direction" refers to a direction perpendicular to one of the end faces.

The height of the scaffold is preferably 0.5 mm or more, more preferably 2 mm or more. The upper limit of the height is not limited and it can be said that it depends on a site where it is used as a prosthetic material. When the height is smaller than 0.5 mm, the scaffold has low mechanical strength and is not preferred as a prosthetic material for a tissue having high mechanical strength such as a knee joint. The scaffold of the present invention can be used to grow a cell on the surface of a prosthetic material by implanting it in a damaged part of a living body. The scaffold can be provided in a desired form.

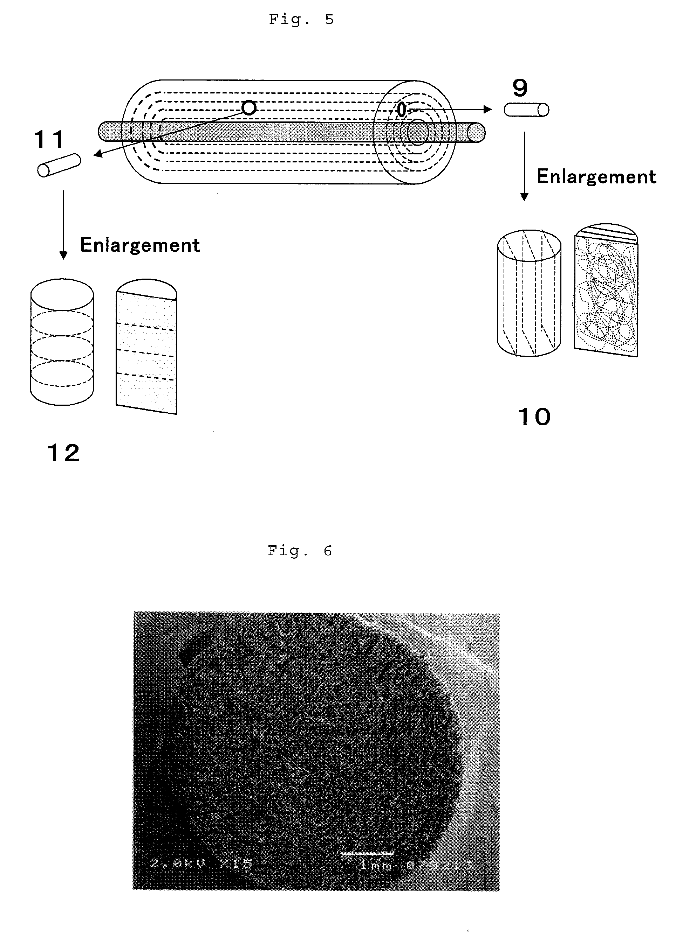

The scaffold of the present invention is composed of an assembly of fibers. In the present invention, the expression "aligned in a plane direction" means that the fibers are aligned substantially parallel to a specific plane. The fibers may be aligned in any one of the transverse, longitudinal and oblique directions as long as they are parallel to this specific plane. The fibers are aligned substantially parallel to the plane shown by broken lines in the cylinder denoted by 10 or 12 in Fig. 5. Parts shown by dotted lines in 10 or 12 of Fig. 5 may form concentric curves. The direction of the fibers on the plane is random.

The fibers are preferably aligned in a plane direction parallel to the height direction as shown by 10 in Fig. 5. Alternatively, as shown by 12 in Fig. 5, the fibers are preferably aligned in a plane direction perpendicular to the height direction.

The diameter of each of the fibers is 0.05 to 50 μm. When the diameter of the fiber is smaller than 0.05 μm, the strength of the scaffold cannot be maintained disadvantageously. When the diameter of the fiber is larger than 50 μm, the specific surface area of the fiber becomes small and the number of living cells decreases. The diameter of the fiber is preferably 0.2 to 50 μm, more preferably 0.2 to 40 μm. The diameter of the fiber can be obtained by observing the scaffold through, for example, a scanning electron microscope (about 200 magnifications).

The arbitrary cross section of the fiber may be substantially spherical or irregular. When the arbitrary cross section of the fiber is irregular, the specific surface area of the fiber increases, whereby the area of the surface of the fiber to which a cell adheres becomes sufficiently large at the time of culture.

The expression "the arbitrary cross section of the fiber is irregular" means that the arbitrary cross section of the fiber has any shape other than a substantially spherical shape and includes a case where the surface of the fiber is roughened to have depressions and/or projections uniformly.

The irregular shape is preferably at least one shape selected from the group consisting of fine depressions on the surface of the fiber, fine projections on the surface of the fiber, depressions formed linearly in the fiber axial direction on the surface of the fiber, projections formed linearly in the fiber axial direction on the surface of the fiber and fine pores on the surface of the fiber. They may be formed alone or in combination. The above "fine depressions" and "fine projections" mean that 0.1 to 1 μm depressions and projections are formed on the surface of the fiber, respectively, and the "fine pores" means that pores having a diameter of 0.1 to 1 μm are existent on the surface of the fiber. The expression "depressions and/or projections formed linearly" means that ribs having a width of 0.1 to 1 μm are formed in the fiber axial direction.

The apparent density of the scaffold is 95 to 350 kg/m$^3$, preferably 100 to 300 kg/m$^3$, more preferably 100 to 250 kg/m$^3$. When the apparent density is lower than 95 kg/m$^3$, mechanical strength becomes low though the intrusion of a cell is satisfactory. When the apparent density is higher than 350 kg/m$^3$, the intrusion of a cell becomes difficult, which is not preferred as a scaffold. The apparent density can be calculated by measuring the volume (area x height) and mass of the obtained assembly.

When the scaffold is used for implantation, mechanical strength high enough to withstand weighted compression in the initial stage of transplantation is required. Shape stability to compression can be provided by aligning the fibers of the scaffold of the present invention in the weighted compression direction. The compressive elastic modulus of the scaffold of the present invention is preferably 0.5 to 5 MPa, more preferably 1.5 to 5 MPa.

The porosity of the scaffold of the present invention is preferably 75 to 90 %, more preferably 78 to 88 %. The porosity is obtained by subtracting the volume of a polymer from the volume of a porous material.

The fibers constituting the scaffold of the present invention are essentially composed of a biocompatible polymer. Each of the fibers comprises a recurring unit derived from a biocompatible monomer in an amount of preferably 80 to 100 mol%, more preferably 90 to 100 mol% of the total of all the recurring units. Examples of the biocompatible monomer include glycolic acid, lactic acid, caprolactones and dioxanones. A blend of biocompatible polymers may also be used.

The biocompatible polymer is preferably a bioabsorbable polymer. The bioabsorbable polymer is preferably essentially composed of an aliphatic polyester. Examples of the aliphatic polyester include polyglycolic acid, polylactic acid, polycaprolactone, polydioxanone, polytrimethylene carbonate, polybutylene succinate, polyethylene succinate and copolymers thereof. Out of these, the aliphatic polyester is preferably at least one selected from the group consisting of polyglycolic acid, polylactic acid, polycaprolactone and copolymers thereof. A copolymer of lactic acid and glycolic acid is particularly preferred. The copolymerization ratio of the former to the latter (mol) is preferably 20/80 to 80/20, more preferably 40/60 to 75/25.

Besides the bioabsorbable polymers, biocompatible polymers such as polyester, nylon, polysulfone, polyurethane, polyethylene, polypropylene, methyl poly(methacrylate), poly(hydroxyethyl methacrylate), poly(vinyl chloride) and polysiloxane may be used as the polymer constituting the porous material.

The intrinsic viscosity of the biocompatible polymer is 0.1 to 1.4 dL/g, preferably 0.04 to 1.3 dL/g, more preferably 0.6 to 1.2 dL/g (30°C, hexafluoroisopropanol).

The scaffold of the present invention may further contain a second component except the biocompatible polymer. The component is preferably at least one selected from the group consisting of cell growth factors such as phospholipids, carbohydrates, glycolipids, steroids, polyamino acids, proteins, polyoxyalkylenes, FGF (fiber blast cell growth factors), EGF (epidermal growth factors), PDGF (platelet-derived growth factors), TGF-β (β type transforming growth factors), NGF (nerve growth factors), HGF (hepatic cell growth factors) and BMP (bone morphogenetic factors). Specific examples of the second component include phospolipids such as phosphatidylcholine, phosphatidylethanolamine, phosphatidylserine and phosphatidylglycerol, and/or carbohydrates such as polygalacturonic acid, heparin, chondroitin sulfate, hyaluronic acid, dermatan sulfate, chondroitin, dextran sulfate, sulfated cellulose, alginic acid, dextran, carboxymethyl chitin, galactomannann, gum Arabic, traganth gum, gellan gum, sulfated gellan, karaya gum, carrageenan, agar, xanthan gum, curdlan, pullulan, cellulose, starch, carboxymethyl cellulose, methyl cellulose, glucomannan, chitin, chitosan, xyloglucan and lenthinan, and/or glucolipids such as galactocerebroside, glucocerebroside, globoside, lactosylceramide, trihexosylceramide, paragloboside, galactosyldiacylglycerol, sulfoquinobosyldiacylglycerol, phosphatidylinositol and glycosylpolyprenol phosphate, and/or steroids such as cholesterols, cholic acid, sapogenin and digitoxin, and/or polyamino acids such as polyaspartic acid, polyglutamic acid and polylysine, and/or proteins such as collagens, gelatin, fibronectin, fibrin, laminin, casein, keratin, sericin and thrombin, and/or polyoxyalkylenes such as polyoxyethylene alkyl ether, polyoxyethylene propylene alkyl ether, polyoxyethylene sorbitan ether. The preferred content of the second component is 0.01 to 50 parts by weight based on 100 parts by weight of the biocompatible polymer.

<manufacturing process>

The scaffold of the present invention can be manufactured through first to third steps.

(first step)

The first step is a so-called "electrospinning method". The first step is to form fibers by delivering a dope containing a biocompatible polymer into an electrostatic field formed between electrodes from a nozzle.

The electrostatic field is formed between a pair of electrodes or among a plurality of electrodes. The electrodes may be made of a metal, inorganic or organic material as long as they show conductivity. Also they may have a conductive metal, inorganic or organic thin film on an insulating material. High voltage may be applied to any one of the above electrodes. The present invention includes a case where two high-voltage electrodes which differ from each other in voltage value (for example, 15 kV and 10 kV) and one electrode connected to an earth are used and a case where more than 3 electrodes are used. Preferably, one of the electrodes is a nozzle and the other electrode is a collection electrode.

The distance between the electrodes which depends on the amount of charge, the size of the nozzle, the flow rate of a spinning liquid and the concentration of the spinning liquid is suitably 5 to 20 cm at 10 kV. The potential of static electricity to be applied is preferably 3 to 100 kV, more preferably 5 to 50 kV, much more preferably 5 to 30 kV.

The dope contains a biocompatible polymer and a solvent. The biocompatible polymer has already been described above. The content of the biocompatible polymer in the dope is preferably 1 to 30 wt%, more preferably 2 to 20 wt%. When the content of the biocompatible polymer is lower than 1 wt%, it is difficult to form fibers disadvantageously. When the content is higher than 30 wt%, the diameter of the obtained fibers becomes too large disadvantageously.

The solvent is preferably a substance which dissolves the biocompatible polymer, has a boiling point of 200°C or lower at normal pressure and is liquid at room temperature. Examples of the solvent include methylene chloride, chloroform, acetone, methanol, ethanol, propanol, isopropanol, toluene, tetrahydrofuran, 1,1,1,3,3,3-hexafluoroisopropanol, water, 1,4-dioxane, carbon tetrachloride, cyclohexane, cyclohexanone, N,N-dimethylformamide and acetonitrile. Out of these, methylene chloride, chloroform and acetone are particularly preferred from the viewpoints of the solubility of a biocompatible polymer, especially an aliphatic polyester. These solvents may be used alone or in combination. In the present invention, another solvent may be used in limits not prejudicial to the object of the present invention.

The diameter of the nozzle is preferably 0.6 to 1.5 mm. When the dope is supplied into the electrostatic field from the nozzle, a plurality of nozzles may be used to increase the production rate of a fibrous material.

Delivery may be carried out by extruding the dope with a syringe having a piston like an injector. A tube having a nozzle at the end may also be used. In this case, the dope is drawn by the potential difference of static electricity to be spun toward the electrode. The fibers may be in a state that the solvent is distilled off or in a state that the solvent is still contained.

A static electricity removing apparatus is preferably used between the nozzle and the electrode. The static electricity removing apparatus is an apparatus which applies an ion air to the fibers to keep ion balance uniform and disperses the fibers into air by easing the charged state of the fibers before they reaches the electrode. The thickness of a roll can be increased by using this apparatus. A scaffold having a large diameter can be obtained by increasing the thickness of the roll.

(second step)

The second step is to wind up the obtained fibers on a rotary winding shaft and accumulate them so as to obtain a roll in which the fibers are aligned in a plane parallel to the winding shaft.

The fibers are accumulated around the winding shaft of a winder between the nozzle and the collection electrode. The shape of the winding shaft may be columnar or prismatic.

Since the average apparent density of the scaffold depends on the revolution of the winding shaft, a scaffold having a desired average apparent density can be obtained by controlling the revolution of the winding shaft. Stated more specifically, when the revolution is high, the average apparent density of the obtained scaffold is high. When the revolution is low, the average apparent density of the obtained scaffold is low. The revolution of the winding shaft is preferably 1 to 1,000 rpm, more preferably 5 to 200 rpm.

The obtained roll is substantially aligned in a plane parallel to the winding shaft. The shape of the roll is arbitrary such as columnar or spindle-like.

After the second step, the obtained roll is preferably heated. The heating temperature is preferably 40 to 90°C. By heating, the fibers are thermally fused to one another, thereby making it possible to obtain a scaffold having excellent compressive strength.

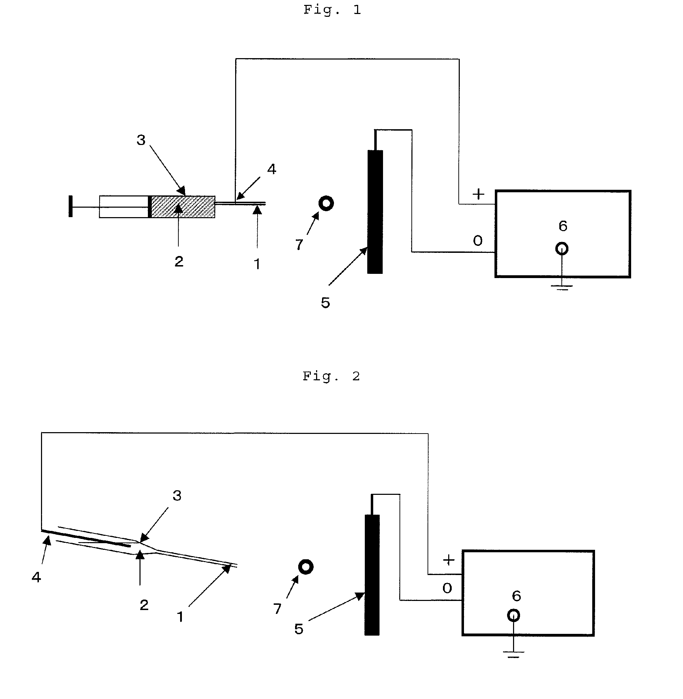

The first step and the second step will be explained with reference to Figs. 1 to 4. Fig. 1 shows an example using a syringe and Fig. 2 shows an example using a tubular discharger. In Fig. 1, the dope (2) is charged into the dope holding tank (3) of a syringe having a nozzle (1). Voltage is applied to the nozzle (1) by a high voltage generator (6) and an electrostatic field is formed between a positive electrode (4) and a negative electrode (5). When the dope (2) is extruded from the nozzle (1), the charged dope is moved toward the negative electrode (5) through the electrostatic field to form fibers.

The fibers can be formed by the method shown in Fig. 2. The dope (2) is charged into the dope holder tank (3) of a tubular discharger having a nozzle (1). A positive electrode (4) is inserted into the dope holding tank (3) and voltage is applied to the nozzle by the high-voltage generator (6) to form an electrostatic field between the positive electrode (4) and the negative electrode (5). The charged dope is discharged from the nozzle (1) by adjusting the distance between the nozzle (1) at the end of the discharger and the negative electrode (5) and moved toward the negative electrode (5) through the electrostatic field to form fibers. The fibers are wound up by the winder (7) before the negative electrode (5).

In the present invention, while the dope (2) is spun toward the negative electrode (5), the solvent is evaporated and the fibers are formed. Although the solvent evaporates completely at normal room temperature while the fibers are collected to the winder (7), if the evaporation of the solvent is incomplete, the dope may be spun under reduced pressure. The spinning temperature depends on the evaporation behavior of the solvent and the viscosity of a spinning liquid but is generally 0 to 50°C.

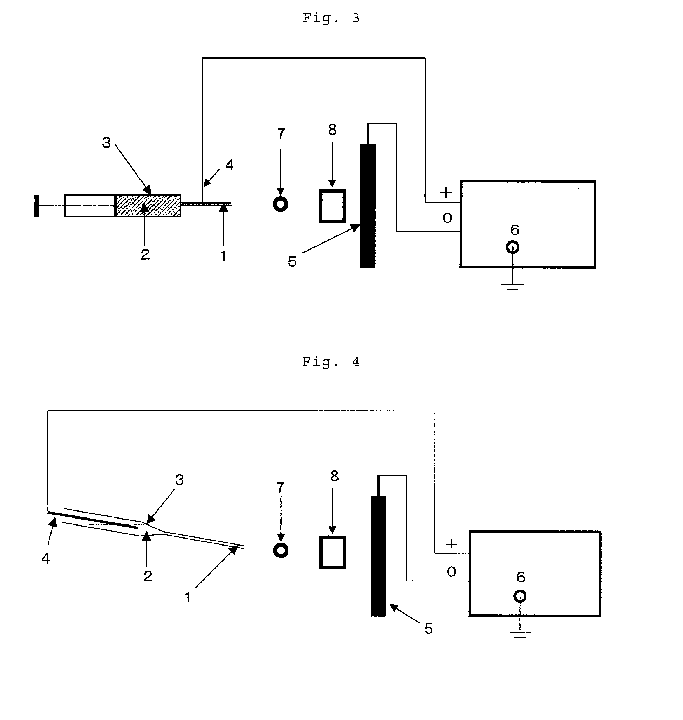

Fig. 3 and Fig. 4 show examples in which a static electricity removing apparatus (8) is installed. The static electricity removing apparatus (8) is installed between the nozzle (1) and the negative electrode (5) to carry out spinning so that the fibers can be collected to the winder (7).

(third step)

The third step is to cut out a 3-D structure from the obtained roll. The 3-D structure can be bored out by using a cylindrical borer as shown in Fig. 5. In Fig. 5, the 3-D structure is preferably bored out (10) such that the height direction of the 3-D structure becomes parallel to the aligning direction of the fibers. Also, the 3-D structure is preferably bored out (12) such that the height direction of the 3-D structure becomes perpendicular to the aligning direction of the fibers. The scaffold (10) is superior in compressive strength to the scaffold (12).

<differentiation and growth of cell>

A cell can be differentiated and grown by using the scaffold of the present invention. The differentiation and growth of a cell may be carried out in vitro or in *vivo. In vitro,* the scaffold may be used as a culture medium for differentiating and growing a cell. *In* vivo, the scaffold of the present invention may be used as a prosthetic material. Particularly, a living tissue can be regenerated by burying the scaffold of the present invention in a damaged affected part. Alternatively, the scaffold of the present invention having a cultured cell in vitro may be buried in an affected part as a prosthetic material. The living tissue is, for example, an osteochondral tissue.

Examples

Materials and measuring methods used in Examples are given below.

(1) Lactic acid-glycolic acid copolymer; LACTEL (DL lactic acid/glycolic acid copolymer, molar ratio = 50/50, intrinsic viscosity: 1.05 dL/g, 30°C, hexafluoroisopropanol, manufactured by Absorbable Polymers International Co., Ltd.)
(2) Methylene chloride, ethanol, formaldehyde; manufactured by Wako Pure Chemical Industries, Ltd.
(3) Rat mesenchymal stem cell; manufactured by Dainippon Sumitomo Pharmaceuticals, Ltd.
(4) MEM (Minimum Essential Medium), FBS(Fetal Bovine Serum), PBS (Phosphate-buffered Saline), antibiotic-antimycotic, 0.05 % Trypsin-EDTA solution; manufactured by Invitrogen Co., Ltd.
(5) L-ascorbic acid 2-phosphate magnesium salts n-hydrate (water content of 26.7 %), β-glycerophosphate disodium n-hydrate, Dexamethason, Triton X-100, Toluidine Blue; manufactured by Sigma Co., Ltd.
(6) Pico Green (registered trademark) ds DNA Quantitation Kit; manufactured by Molecular Probe Co., Ltd.

<Example 1>

(preparation of dope)

A lactic acid-glycolic acid copolymer (molar ratio= 50/50) was dissolved in a mixed solvent of methylene chloride and ethanol to prepare a 15 wt% dope.

(spinning)

A cylindrical roll composed of an assembly of fibers was obtained by the electrospinning method using the apparatus shown in Fig. 4. The inner diameter of the nozzle (1) was 1.3 mm. The distance from the nozzle (1) to the winder (7) was 20 cm, and the distance from the nozzle (1) to the static electricity removing apparatus (8) was 35 cm. Applied voltage was 15 kV. The winder (7) and the static electricity removing apparatus (8) manufactured by Kasuga Denki Co., Ltd. were installed between the nozzle (1) and the negative electrode (5). The positive electrode (4) was inserted into the dope holding tank (3). The revolution of the winder (7) was set to 100 rpm.
The dope was fed to the dope holding tank (3), the distance between the nozzle (1) and the negative electrode (5) was adjusted, and fibers were delivered from the nozzle (1). Delivery was continued for 120 minutes, and the fibers were wound up by the rotating winder (7) to obtain a cylindrical roll. The roll was put into a thermostatic device and heated at 80°C for 10 minutes.

(cutting out)

A cylindrical scaffold having a diameter of 5 mm and a height of 5 mm denoted by 10 in Fig. 5 was cut out from the obtained roll by using a biopsy trepan (manufactured by Kai Industries, Ltd.) as shown in Fig. 5.

(evaluation of characteristic properties)

The characteristic properties of the obtained scaffold were measured by the following methods. The results are shown in Table 1.

(1) diameter of fiber

The diameter of each fiber was observed visually through a digital microscope (VHX Digital Microscope of Keyence Co., Ltd.) or a scanning electron microscope (manufactured by JEOL Ltd., 200 magnifications). Arbitrary 10 fibers were selected from each view field in electron microscopic observation and measured, and this operation was carried out for 5 view fields to calculate the average value of 50 fibers. Massive foreign matter or a bundle of fibers fused to one another produced in the step of forming fibers were not measured.

(2) apparent density of scaffold

The apparent density of the scaffold was calculated from the following equation.

$$\rho = 4m/\pi d^2 h$$

(p: apparent density of porous material, m: mass, d: diameter, h: height)

(3) porosity of scaffold

The porosity of the scaffold was calculated from the following equation.

$$\varepsilon = 1 - \rho/\rho_0$$

($\varepsilon$: porosity of scaffold, p: apparent density of porous material, $\rho_0$: intrinsic density of polymer)

(4) compressive strength

The compressive strength of the scaffold corresponding to (10) in Fig. 5 was measured in accordance with JISK 7220. That is, the scaffold in which fibers were aligned parallel to the height direction of the cylinder was measured. A test specimen was placed between the pressure planes of a material tester, the center line of the specimen was aligned with the center lines of the pressure planes, and it was confirmed that the upper and lower surfaces of the specimen were parallel to the pressure planes. A load was applied to the test specimen at a constant test speed of 10 mm/min to measure compressive strength until a compression limit was reached.

<Example 2>

(preparation of dope)

The same lactic acid-glycolic acid copolymer as in Example 1 was used to prepare a 10 wt% dope.

(spinning)

A cylindrical roll composed of an assembly of fibers was obtained in the same manner as in Example 1 except that the delivery time was set to 90 minutes and the heat treatment was carried out at 70°C for 10 minutes.

(cutting out)

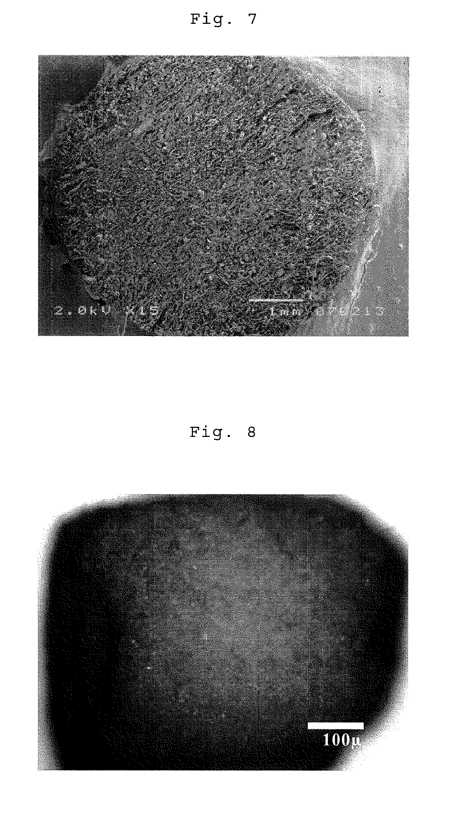

A cylindrical scaffold having a diameter of 5 mm and a height of 5 mm corresponding to 10 in Fig. 5 was cut out from the obtained roll in the same manner as in Example 1. Fig. 6 (top end face) and Fig. 7 (bottom end face) show microphotographs (15 magnifications) of a section parallel to the end faces of the scaffold corresponding to 10 in Fig. 5. It is seen that the fibers were accumulated in layers.

(evaluation of characteristic properties)

The characteristics properties of the scaffold were evaluated in the same manner as in Example 1. The results are shown in Table 1.

(biological evaluation of scaffold)

(preparation of cell)

The biological evaluation of the scaffold was carried out by the following method. The mesenchymal stem cell of a rat was cultured in MEM containing 15 % of FBS and 1 % of antibiotic-antimycotic at 37°C in a 5 % $CO_2$ atmosphere

for 3 passages.

(sowing and culture of cell)

The prepared rat mesenchymal stem cells were seeded in the obtained scaffold at a density of 6.0 x $10^6/cm^3$ and cultured in MEM containing 15 % of FBS, 1 % of antibiotic-antimycotic, 10 $\mu$M of dexamethasone, 50 $\mu$M of L-ascorbic acid 2-phosphate magnesium salts n-hydrate and 10 mM of $\beta$-glycerophosphate disodium n-hydrate at 37°C in a 5 % $CO_2$ incubator for 12 days. The culture medium was exchanged 3 times a week.

(evaluation)

The scaffold was taken out on the first day, sixth day and 12-th day of culture to measure the amount of DNA and evaluate it histologically.

(1) Amount of DNA
The amount of DNA was measured based on the measurement manual of Pico Green (registered trademark) ds DNA Quantitation Kit. The sample to be measured was frozen and molten with 0.2 % of Triton-X100 three times and ground with supersonic waves to obtain a cell suspension, and an extract was prepared from the suspension. 100 $\mu$l of the measured sample treated with enzyme was put into a micro-plate with 96 holes, and 100 $\mu$l of Pico Green (registered trademark) ds DNA Quantitation Reagent diluted with TE (pH of 7.5) 200 times was added to the sample to measure the amount of DNA with 485 nm excitation light and 535 nm fluorescence. A calibration curve was drawn from the value of a standard DNA solution and the amount of DNA of the measurement sample was calculated based on the curve. The result is shown in Table 2. The amount of DNA is proportional to the number of grown cells.
(2) Histological evaluation
For histological evaluation, the scaffold was immersed in 10 % of formaldehyde before sampling. Before it was stained, it was cleaned with distilled water, immersed in 100 % of ethanol for 1 hour twice, 90 % of ethanol for 1 hour and 70 % of ethanol for 1 hour to be cleaned while it was diluted stepwise. The obtained scaffold was immersed in distilled water for 15 minutes to be cleaned and then in a 0.4 % toluidine blue-aqueous solution for 1 minute. Thereafter, it was cleaned in running water for 1 minute to remove excess of a staining solution and observed through a digital microscope at 450 magnifications.
(3) Result

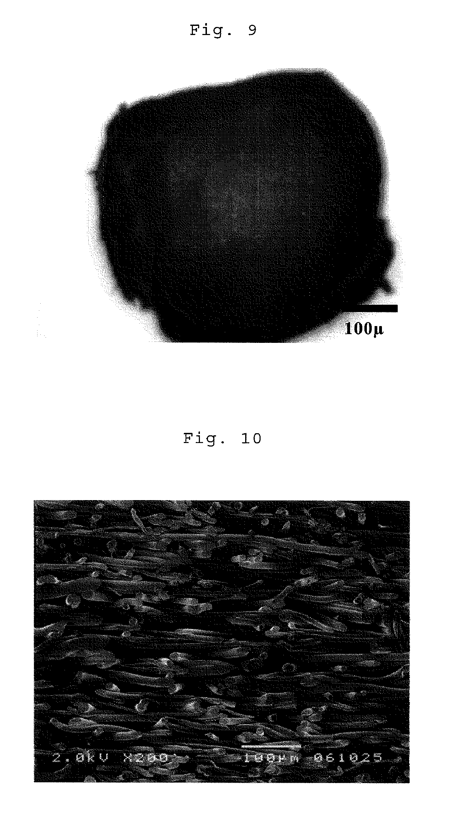

The measurement result of the amount of DNA is shown in Table 2. Fig. 8 and Fig. 9 show photomicrographs of the stained scaffold on the 1st and 12-th days of culture. It is seen that the scaffold on the 12-th day of culture has higher staining density than the scaffold on the 1st day, the stained area reaches a deep portion of the scaffold, and the growth of the cell and the production of a cartilage matrix proceed well.

<Example 3>

(manufacture of scaffold)

The operation of Example 2 was repeated to obtain a cylindrical scaffold having a diameter of 5 mm and a height of 5 mm corresponding to 12 in Fig. 5. The measurement results of the characteristic properties of the scaffold are shown in Table 1.

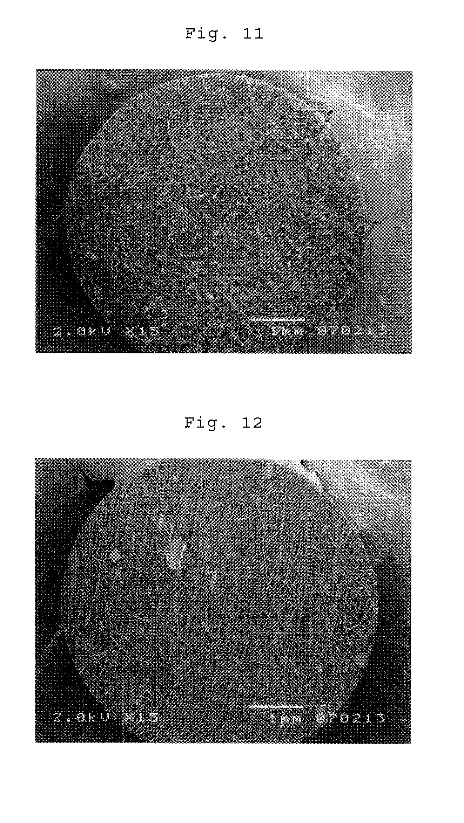

Fig. 10 shows a photomicrograph (200 magnifications) of a section perpendicular to the end surface of the scaffold corresponding to 12 in Fig. 5. It is seen that the fibers are aligned in a plane direction. Fig. 11 (top end surface) and Fig. 12 (bottom end surface) show photomicrographs (15 magnifications) of a section parallel to the end faces of the scaffold corresponding to (12) in Fig. 5. It is seen that the fibers are accumulated densely like the mesh of a net.

(biological evaluation)

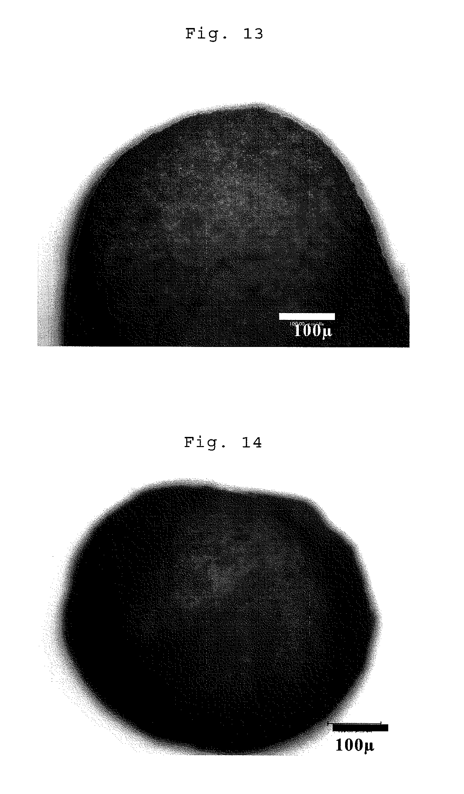

A cell was cultured in the same manner as in Example 2 except that the obtained scaffold was used to measure the amount of DNA. The result is shown in Table 2. Fig. 13 and Fig. 14 show photomicrographs of the stained scaffold on the 1st and 12-th days of culture. It is seen that the scaffold on the 12-th day of culture has higher staining density than the scaffold on the 1st day, the stained area reaches a deep portion from the surface of the scaffold, and the growth of a cell and the production of a cartilage matrix proceed well like Example 2.

Table 1

| | Spinning conditions | | Diameter of fiber (μm) | Apparent density (kg/m³) | Porosity (%) | Compressive strength | |
|---|---|---|---|---|---|---|---|
| | Revolution (rpm)*1 | Time (min) | | | | Compressive elastic modulus (MPa) | 10 % displacement stress (MPa) |
| Example 1 | 100 | 120 | 5 | 220 | 83.5 | 2.61 | 0.99 |
| Example 2 | 100 | 90 | 4.6 | 214 | 83.7 | 4.58 | 0.12 |
| Example 3 | 100 | 90 | 7.8 | 224 | 82.0 | 1.34 | 0.01 |
| *1: revolution of winder (rpm) | | | | | | | |

Table 2

| | Time change in amount of DNA (μg/scaffold) | |
|---|---|---|
| | Example 2 | Example 3 |
| 1st day of culture | 0.93 | 0.89 |
| 6-th day of culture | 1.02 | 1.06 |
| 12-th day of culture | 1.09 | 0.87 |

Effect of the Invention

The scaffold of the present invention has such high mechanical strength that it can withstand weighted compression in the initial stage of transplantation. Therefore, it can be used in a site which requires mechanical properties, such as a cartilage damaged part. Since the scaffold of the present invention is composed of a biocompatible polymer, it has no bad influence upon a living body. The scaffold of the present invention has a certain fiber density, facilitates the intrusion of a cell and enables the supply of oxygen and nutrition and the discharge of carbon dioxide and waste matter to be carried out swiftly. Therefore, the scaffold can grow a cell well.

According to the manufacturing process of the present invention, the scaffold can be manufactured easily. According to the manufacturing process of the present invention, as fibers obtained by the electrospinning method are accumulated on a rotary shaft, accumulated fibers having a uniform accumulation density in the accumulation direction are obtained. Accumulated fibers uniform in a plane parallel to the winding shaft are obtained. Further, certain tension is applied to the fibers by winding up the fibers on the rotary winding shaft, thereby making it possible to obtain high-density accumulated fibers.

According to the cell growing method of the present invention, a cell can be grown well. According to the living tissue regeneration method of the present invention, a damaged living tissue can be regenerated well.

Industrial Applicability

The scaffold of the present invention is useful as a cell culture medium in the field of regeneration medicine. The scaffold of the present invention is useful as a prosthetic material, especially a prosthetic material for a site in which mechanical properties are important, such as an osteochondral damaged part.

**Claims**

1. A scaffold composed of an assembly of fibers and having a 3-D structure consisting of two end faces and a side face, wherein

(1) the fibers are aligned in a plane direction;
(2) the fibers have a diameter of 0.05 to 50 μm;
(3) the fibers are essentially composed of a biocompatible polymer; and

(4) the scaffold has an apparent density of 95 to 350 kg/m$^3$.

2. The scaffold according to claim 1, wherein the fibers are aligned in a plane direction parallel to the height direction.

3. The scaffold according to claim 1, wherein the fibers are aligned in a plane direction perpendicular to the height direction.

4. The scaffold according to claim 1, wherein the fibers have a diameter of 0.2 to 40 $\mu$m.

5. The scaffold according to claim 1, which has an apparent density of 100 to 250 kg/m$^3$.

6. The scaffold according to claim 1, which has a porosity of 75 to 90 %.

7. The scaffold according to claim 1, which has a height of 0.5 mm or more.

8. The scaffold according to claim 1, which has an end face area of 0.05 to 8 cm$^2$.

9. The scaffold according to claim 1, which is cylindrical or polygonal column-like.

10. The scaffold according to claim 1, which has a compressive elastic modulus in the height direction of 0.5 to 5 MPa.

11. The scaffold according to claim 1, wherein the biocompatible polymer is bioabsorbable.

12. The scaffold according to claim 1, wherein the biocompatible polymer is an aliphatic polyester.

13. The scaffold according to claim 1, wherein the aliphatic polyester is at least one selected from the group consisting of polyglycolic acid, polylactic acid, polycaprolactone and copolymers thereof.

14. The scaffold according to claim 1, which is a prosthetic material or a cell culture medium.

15. A process of manufacturing the scaffold of any one of claims 1 to 12, comprising the steps of:

(1) delivering a dope containing a biocompatible polymer into an electrostatic field formed between electrodes from a nozzle to form fibers;
(2) winding up the obtained fibers on a winding shaft to form a roll in which the fibers are aligned in a plane direction parallel to the winding shaft; and
(3) cutting out a 3-D structure from the obtained roll.

16. The manufacturing process according to claim 14, wherein the fibers are formed by using a static electricity removing apparatus.

17. A method of differentiating and growing a cell by using the scaffold of claim 1.

18. A method of regenerating a living tissue by burying the scaffold of claim 1 into a damaged affected part.

Fig. 1

Fig. 2

Fig. 3

Fig. 4

Fig. 5

11    Enlargement

12

9    Enlargement

10

Fig. 6

2.0kV X15    1mm 070213

Fig. 7

Fig. 8

Fig. 9

100μ

Fig. 10

Fig. 11

Fig. 12

Fig. 13

100μ

Fig. 14

100μ

## INTERNATIONAL SEARCH REPORT

| International application No. |
|---|
| PCT/JP2007/054739 |

A. CLASSIFICATION OF SUBJECT MATTER
*A61L27/00*(2006.01)i, *C12M3/00*(2006.01)i, *C12N5/06*(2006.01)i, *D04H1/72* (2006.01)i

According to International Patent Classification (IPC) or to both national classification and IPC

B. FIELDS SEARCHED

Minimum documentation searched (classification system followed by classification symbols)
A61L27/00, C12M3/00, C12N5/06, D04H1/72

Documentation searched other than minimum documentation to the extent that such documents are included in the fields searched
Jitsuyo Shinan Koho           1922-1996   Jitsuyo Shinan Toroku Koho   1996-2007
Kokai Jitsuyo Shinan Koho     1971-2007   Toroku Jitsuyo Shinan Koho   1994-2007

Electronic data base consulted during the international search (name of data base and, where practicable, search terms used)
CAPlus/BIOSIS/MEDLINE/EMBASE(STN), JSTPlus/JMEDPlus/JST7580(JDream2)

C. DOCUMENTS CONSIDERED TO BE RELEVANT

| Category* | Citation of document, with indication, where appropriate, of the relevant passages | Relevant to claim No. |
|---|---|---|
| X | MATTHEWS,J.A. et al, Electrospinning of Collagen Nanofibers, Biomacromolecules, 2002, Vol.3, No.2, p.232-238 | 1-11,14-18 |
| X | BOLAND,E.D. et al, Electrospinning of bioresorbable polymers for tissue engineering scaffolds, ACS Symposium Series, Feb. 2006, Vol.918 (Polymeric Nanofibers), p.188-204 | 1-18 |

☒  Further documents are listed in the continuation of Box C.        ☐  See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 02 April, 2007 (02.04.07) | 17 April, 2007 (17.04.07) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.

PCT/JP2007/054739

| C (Continuation). DOCUMENTS CONSIDERED TO BE RELEVANT | | |
|---|---|---|
| Category* | Citation of document, with indication, where appropriate, of the relevant passages | Relevant to claim No. |
| X | JP 51-040476 A (IMPERIAL CHEM IND LTD),<br>05 April, 1976 (05.04.76),<br>Full text; particularly, Fig. 3<br>& DE 2534935 A      & SE 7508781 A<br>& FR 2281448 A      & NL 7512279 A<br>& US 4043331 A      & US 4044404 A<br>& GB 1527592 A      & GB 1530990 A<br>& IT 1042990 B      & IT 1044659 B<br>& CA 1090071 A      & NL 183835 B<br>& US 4878908 A | 1-11,14-18 |

Form PCT/ISA/210 (continuation of second sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 200488024 A

- JP 2004520855 A